# EUROPEAN PATENT APPLICATION

(11) **EP 4 596 001 A1**
(43) Date of publication of application: **06.08.2025**
(21) Application number: 24155509.3
(22) Date of filing: 02.02.2024
(51) Int. Cl.: A61M 5/315

(54) **MAGNIFYING LENS FOR A DRUG DELIVERY DEVICE**

(71) Applicant: Ypsomed AG, 3401 Burgdorf (CH)
(72) Inventor: BOSSHARD, Simon Martin, 3324 Hindelbank (CH); HIRSCHEL, Jürg, 3007 Bern (CH); VON MÜHLENEN, Beat, 3043 Uetligen (CH)
(74) Representative: Kleiner, Stefan

(57) **Abstract**

The invention relates to a dose setting mechanism for a drug delivery device (1) for dispensing a liquid drug from a reservoir (3) through an injection needle. The dose setting mechanism is includes a housing (10) having a central axis and comprising a lateral opening (11) and an indication member (30) movable relative to the housing and comprising an indication (32) representing a dose value. The indication (32) is visible from outside through the opening (11). The dose setting mechanism further includes a magnifying lens (52) adapted to provide a magnified view of the indication (32) and positioned in the second lateral opening (21) and integrated in the insert (20) wherein a maximum radial dimension measured from the central axis to an outer surface of the magnifying lens is equal or less than a minimum radial dimension from the central axis to an inner surface of the housing (10).

## Description

### FIELD OF THE INVENTION

The present invention relates to a dose setting mechanism for a drug delivery device for injecting, delivering, administering, infusing or dispensing substances and/or liquids such as insulin, hormone preparations or vaccines. It departs from a dose setting mechanism comprising a housing with a lateral opening and an indication member movable relative to the housing during dose setting or dispensing and including an indication representing a dose value, wherein the indication is visible from outside through the opening.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

There are several types of drug delivery devices known in the art. Disposable delivery devices are adapted to deliver medication from a container such as a pre-filled cartridge or syringe that is not intended to be replaced or refilled by the patient. Reusable, semi-disposable, or hybrid delivery devices have components that may be replaced by the patient, or a cartridge that may be refilled, while some components of the device may be reused with the replaced or refilled drug container.

In order to facilitate a manipulation and in particular a setting or correcting a dose for users with visual impairment approaches exist that may improve the visibility and readability.

For example, EP0937477 A2 discloses an injection pen with a housing comprising a longitudinally extending viewing port through which an outer surface of a dose barrel can be seen. Through the viewing port a plurality of dosage numerals printed on the dose barrel is visible to a user. The viewing port in the housing incorporates a magnifying lens to enlarge dosage numeral to increase readability of these numerals.

EP1332767 A1 discloses an injector comprising a dosage drum and a window through which a scale indicating the dose can be read. The window includes a magnifying lens.

US2013018311 A1 discloses an injection pen with a housing and a cartridge holder connectable to the housing. The cartridge holder has a side wall which includes a Fresnel lens integrally formed with the side wall. The side wall and Fresnel lens are made from a clear resin such as polypropylene or polycarbonate. The Fresnel lens includes a plurality of lens zones and is provided to magnify the graduations on a cartridge held by the cartridge holder to facilitate determining the volume of medicinal fluid within the fluid chamber of the cartridge.

### DESCRIPTION OF THE INVENTION

It is an objective of the invention to retrofit an improved readability for a dosage scale in an existing drug delivery device.

This objective is achieved by a dose setting mechanism or a drug delivery device according to the independent claims. Preferred embodiments are evident from the dependent claims.

According to the invention a dose setting mechanism with a magnifying lens is disclosed herein. The dose setting mechanism is used for a drug delivery device for dispensing a liquid drug from a reservoir through an injection needle or cannula. The dose setting mechanism is adapted to set a dose to be dispensed from the reservoir and comprises
- a sleeve-shaped housing defining a longitudinal direction and having a central axis and comprising a first lateral opening in a side wall of the sleeve-shaped housing;
- a threaded insert arranged inside the housing and comprising a second lateral opening in a side wall of the insert wherein the second opening matches with the first lateral opening and wherein the insert is fixedly and non-movably connected to the housing and wherein the insert comprises an internal thread on an inner side of the insert;
- an indication member comprising an indication on an outer surface representing a dose value, wherein the indication is visible from outside through the first and second lateral opening and wherein the indication member comprises an outer thread adapted to cooperate with the internal thread of the insert;
- a magnifying lens adapted to provide a magnification or magnified view of the indication.

The dose setting mechanism is characterized in that the magnifying lens is positioned in the second lateral opening and integrated in the insert or enclosed by the insert, wherein a maximum radial distance measured from the central axis to an outer surface of the magnifying lens is equal or less than a minimum radial distance from the central axis to an inner surface of the housing surrounding the insert.

With respect to the insert that means a maximum radius from the central axis to an outer surface of the insert is equal or less than a maximum radius from the cental axis to an outer surface of the magnifying lens. In other words, a maximum cross-sectional area of the insert including the lens does not exceed a minimal internal cross-sectional area of the housing. The housing may have a circular or non-circular cross-section.

That has the advantages that the magnifying lens together with the insert can be inserted into the housing for assembly which allows a retrofit of the magnifying lens to an already existing housing and existing insert. That is, an already designed and existing housing and insert do not have to be modified to be able to add a magnifying lens to the dose setting mechanism. That provides a cost-saving retrofit of the dose setting mechanism.

Furthermore, as the magnifying lens is connected to the threaded insert the lens and the insert can be mounted to a preassembled unit which may facilitate the final assembly when the threaded insert is connected to or/and inserted into the housing.

The indication member is movable to set a dose, correct a dose or/and dispense a dose. It is of significant importance that the user can clearly read an indication, namely characters, signs, a code or markings on an outer surface of the indication member to correctly set a dose, correct a dose or/and dispense a dose. The magnifying lens allows for a better visibility and readability of the indication as the magnifying lens provides an enlarged and/or clear and improved depiction of the indication.

According to the invention the magnifying lens is integrated in or enclosed by the insert. That means the insert encompasses the magnifying lens, for example by a wall or by a retaining structure.

The threaded insert is arranged inside the sleeve-shaped housing or the housing may surround the insert. The insert may be, for example, a sleeve-shaped member coaxially positioned inside the housing or the insert may be a nut or half-nut connected to the sleeve-shaped housing, preferably connected to an inside of the housing. The insert comprises an opening or a cavity with an inner wall which includes an inner thread adapted to cooperate with an external thread of the indication member. The latter is coaxially (or radially) arranged inside the insert and movable relative to the insert due to the threaded engagement.

The threaded insert has a second lateral opening which is located radially inside to the first lateral opening of the housing and matches with the first opening such that a user can view through the first and second lateral openings from outside through the housing and to the indication member.

The indication member is movable (rotatable, linearly movable or a combination thereof) relative to the housing in order to set a dose, correct a dose or during dose dispensing. The indication member may be coupled or connected to a dose set member or it may be monolithic formed with the dose set member such as, for example, a dose sleeve, a dose knob or dose button.

The indication member may be, for example, a sleeve or display element comprising the indication on an outer surface that is visible by the user through the lateral opening in the outer housing. The indication preferably represents a dose value and may comprise, for example, a digit or digit sequence, a number, a letter, color markings or human or computer readable codes such as, for example, QR codes or grey codes. When the indication member is moved relative to the housing the indication visible through the opening alters. That means at a specific indication member position a specific indication is visible through the opening for the user.

The indication member may be, for example, a sleeve or display element comprising the indication on an outer surface that is visible by the user through the lateral opening in the outer housing. The indication preferably represents a dose value and may comprise, for example, a digit or digit sequence, a number, a letter, color markings or human or computer readable codes such as, for example, QR codes or grey codes. When the indication member is moved relative to the housing the indication visible through the opening alters. That means at a specific indication member position a specific indication is visible through the opening for the user.

In a preferred embodiment the dose setting mechanism is used in a drug delivery device and in particular in an injection device. The term "injection device" or "injector" refers to a device that is removed from the injection site after each medication event or drug delivery process. By contrast, an "infusion device" refers to a device with a cannula or needle that remains in the skin of the patient for a prolonged period of time, for example, several hours.

The injection device is preferably a pen-shaped injection device. In a preferred embodiment the injection device is a disposable injection device. Alternatively, the injection device may be a reusable or semi-reusable device. A semi-disposable injection device comprises components or sub-assemblies that are disposed of after an injection or once the medication is fully dispensed. Additionally, it comprises further components or sub-assemblies that can be reused (reusable part) for several injections and successively with several identical disposable parts.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

Preferably, the magnifying lens has an arched-shaped outer contour. That enables the magnifying lens to optimally use the cavity or space between an outer surface of the insert and an inner surface of the sleeve-shaped housing in an assembled state or the space between an opening in the insert and the inner surface of the housing. This space in between the insert and the housing is preferably also arched-shaped or is the shape of a circular segment in a cross-section. In other words, the shape or form of the space in a plane perpendicular to the longitudinal axis of the housing is arched-shaped or is a circular segment. Thus, the space can be used and filled in an optimal manner and the magnifying lens can be design as large as possible to provide an optimal magnification.

In a further preferred embodiment, the threaded insert comprises an integrated holding portion or holding structure integrally formed in the insert and arranged in the area of the second lateral opening of the insert and adapted to hold the magnifying lens in place. The holding portion is integrally formed with the insert and is hence monolithic as one-piece with the rest of the insert and made, for example, by plastic injection molding.

The holding portion is adapted to fix and hold the magnifying lens such that the magnifying lens is not movable relative to the threaded insert. The holding portion may comprise, for example, clamps, retaining cams or ledges or it may be a frame, enclosure or circumferential wall enveloping or enclosing the magnifying lens.

The holding portion is arranged next to or at least partially around the lateral opening of the insert such that the magnifying lens can be connected to the insert above or inside the second opening allowing the user to view through the magnifying lens and through the second opening towards the indication member.

Preferably, the holding portion comprises is a collar or wall at least partially encompassing the magnifying lens such that the magnifying lens is axially and radially held in place by a form fit. The form fit provides an easy assembly as the magnifying lens can be inserted in the collar or wall and can snap into place.

Alternatively, the lens can be held by force fit or it can be glued or bonded onto the threaded insert.

In an alternative embodiment the threaded insert may be made monolithic from a transparent material including a lens section providing a magnification. In this embodiment the lens is not a separate element but integrally formed and monolithic with the threaded insert. The transparent material may be a plastic or a composite material.

In a further alternative embodiment the threaded insert is made by two-component injection molding or multi-material injection molding wherein a first component (first material) is used for the internal thread and the body of the insert and a second component (second material) is used for making the magnifying lens. The two-component threaded insert is hence made of two or more different materials (e. g. plastics or a composite material).

The threaded insert is preferably made by a bi-injection molding technique. In this process, two or more different materials are fed into a mold cavity at different points and pressed through different valves. The materials do not physically mix together such that a first component or material for the insert body does not mix with a second component or material which provides the magnification. With such a two-component injection molded threaded insert the number of components to be assembled can be reduced which facilitates the handling and assembly process.

The magnifying lens is preferably a Fresnel lens which comprises a plurality of lens sections (preferably ring-shaped segments and concentrically arranged). The Fresnel lens allows to use a thin lens and the mass and volume of lens material is reduced compared to a lens of conventional design. The Fresnel lens is preferably made of glass or plastic.

In a further embodiment and unlike to above-described embodiment with the threaded insert the Fresnel lens may be directly integrated in the housing and namely arranged in the first lateral opening of the housing.

In an alternative embodiment the magnifying lens may be a conventional lens which is thicker than the Fresnel lens.

Preferably, the Fresnel lens has a flat or planar surface and an uneven (not planar) or structured surface. The uneven surface is preferably directed towards the indication member such that the flat surface is on an outer side. In this case the uneven surface forming the Fresnel effect is protected against working-over or environmental impacts.

In a preferred embodiment a dimension of the lens (conventional lens or Fresnel lens) along the longitudinal axis (length) is larger than a dimension of the lens in a direction perpendicular to the longitudinal axis (width) or larger than a dimension of the lens in a circumferential direction around the longitudinal axis.

A lens that is longer in a first direction than in a second direction may provide a stronger magnification in the first direction than in the second direction. Namely, such a lens can provide an enlarged view of a compressed indication. That means the indication and in particular a character, digit or letter can be printed in a compressed or shrunk design onto the indication member. That allows to print a row or sequence of indications in a space-saving design.

Namely, a lens longer in a first direction than in a second direction can then magnify the compressed indication in the first direction but not in the second direction (or it can magnify more in a first direction than in a second direction) and thus the compressed indication is transformed back to a normal or non-compressed appearance for the user.

That means in a tight spatial environmental the indication can be printed in a compressed shape and due to the lens, the indication is visible for the user in a normal and non-compressed design.

In a preferred embodiment the indication member is a sleeve rotatable relative to the housing for dose setting and preferably additionally for dose correcting. Preferably, the indication sleeve is rotatable in a first direction to set a dose and in a second, counter direction to dispense a dose and/or to correct a set dose.

The indication sleeve is preferably arranged inside the housing and preferably in threaded connection with the housing or with a threaded insert which is fixedly connected to the housing such that the insert cannot be moved relative to the housing.

The housing is preferably the outermost part such that the housing envelops the indication member. The housing may be further adapted to envelop a dispensing mechanism of the delivery device. The housing is further preferably sleeve-shaped which allows to accommodate the dose and/or dispensing mechanism of the drug delivery device.

The invention relates further to a drug delivery device comprising a reservoir holder fixing and holding the reservoir in place relative to the housing. The reservoir holder is releasably (reusable device) or non-releasably (disposable device) attachable to the housing.

Furthermore, the drug delivery device comprises the dispensing mechanism for dispensing the set dose as described above. The dispensing mechanism may comprise a dose set member rotatable relative to the housing to set a dose, a drive member that can be rotationally coupled or decoupled to the dose set member and a plunger rod operatively coupled to the drive member.

During dose setting the drive member is preferably not rotationally coupled to the dose set member such that the dose set member is rotatable relative to the drive member. During dispensing of the set dose, the dose set member may be rotationally coupled to the drive member to transmit a rotation of the dose set member to the drive member to move the plunger rod in dispensing direction. The indication member is preferably coupled (directly or indirectly via intermediate member) to the dose set member such that a rotation of the dose set member is transferred to the indication member and vice versa. Alternatively, the indication member may be monolithic formed with the dose set member and thus formed as a one-piece.

Preferably, the drug delivery device is a pen-shaped injection device and the reservoir holder includes a connecting portion adapted to releasably attach an injection needle and wherein the reservoir is a cartridge. The injection device may be a disposable injection pen or a reusable pen with a reservoir holder that can be releasably attached to the housing.

In addition to the above, a further arrangement with a dose setting mechanism with a magnifying member in a flexible label is disclosed herein. The dose setting mechanism according to this further arrangement can be used in a drug delivery device, preferably an injection device, for dispensing a liquid drug from a reservoir through an injection needle or cannula. The dose setting mechanism is adapted to set a dose to be dispensed from the reservoir. The dose setting mechanism comprises
- a housing defining a longitudinal axis and comprising a lateral opening in the housing;
- an indication member movable relative to the housing and comprising an indication (character, code or marking) representing a dose value, wherein the indication is visible from outside through the opening.

The dose setting mechanism further comprises a flexible label attached to an outer surface of the housing. The flexible label has a magnifying lens integrated in the flexible label or attached or connected to the flexible label. The magnifying lens is positioned in the range of the lateral opening such that the indication can be seen from outside by the user through the magnifying lens.

The indication member is movable to set a dose, correct a dose or/and dispense a dose. It is of significant importance that the user can clearly read an indication, namely characters, signs, a code or markings on an outer surface of the indication member to correctly set a dose, correct a dose or/and dispense a dose. The magnifying lens allows for a better visibility and readability of the indication as the magnifying lens provides an enlarged and/or clear and improved depiction of the indication.

The magnifying lens is integrated or attached to a flexible label which can be attached to an outer surface of the housing. This enables a retrofit and provides an easy and cost-saving manufacturing process.

The flexible label is preferably attached and namely permanently bonded to an outer surface of the housing of the dose setting mechanism or of the drug delivery device.

The flexible label can be attached to the housing. As the label is flexible the label may be adapted or fitted to an outer housing contour. The label and the magnifying lens may be monolithic formed in one-piece or alternatively, the magnifying lens may be integrated or embedded in the flexible label. In the latter case the lens may be rigid or it may be flexible too. When the label is mounted to the housing the magnifying lens is located in the area of the lateral opening, preferably the magnifying lens covers the lateral opening such that the indication member can be seen exclusively through the magnifying lens.

The label preferably includes a bonding section adapted to bond the label onto an outer housing surface and the label includes additionally a lens section wherein the bonding section and the lens section have the same label thickness. That means the complete label has the same thickness which provides a device with compact outer dimensions. Furthermore, a label with a constant thickness across its length may be easier to handle and to attach to the housing.

Preferably, the flexible label and the lens are made of the same material. That means the magnifying lens is monolithic formed with the flexible label as one-piece. In this case the magnifying lens may be made of flexible plastic which has a magnifying portion, for example, with fractures or structured elements providing the magnifying effect.

Alternatively, the flexible label may be made of a two-component injection molded material comprising a first material for a bonding or attachment section of the label and a second material for the lens section of the label.

The following described embodiments include the same features as the described above with respect to the first arrangement with the magnifying lens integrated in the treaded insert. The described functions and advantages of those embodiments apply also to the arrangement with the magnifying lens integrated in the flexible label.

In a preferred embodiment the magnifying lens is a Fresnel lens comprising a plurality of lens sections.

The lens (conventional lens or Fresnel lens) may comprise a flat surface and an uneven surface, wherein the uneven surface is directed towards the indication member.

Preferably, a dimension of the lens along the longitudinal axis is larger than a dimension of the lens in a direction perpendicular to the longitudinal axis.

The indication member may be a sleeve rotatable relative to the housing for dose setting. The housing preferably envelops the indication member and the housing is further adapted to envelop a dispensing mechanism of the delivery device.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of an injection pen as known in the art;
- Fig. 2: depicts a perspective view of the injection pen with a label including a magnifying lens;
- Fig. 3: depicts a sectional view of the injection pen of figure 1;
- Fig. 4: depicts a perspective view of an indication sleeve of the injection pen;
- Fig. 5a: depict a perspective view of a threaded insert with a Fresnel lens;
- Fig. 5b: depicts a view of cross-section of the insert of figure 5a;
- Fig. 5c: depicts an enlarged view of the depiction of the Fresnel lens of figure 5b;
- Fig. 6a, 6b: depict the insert with a conventional lens.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

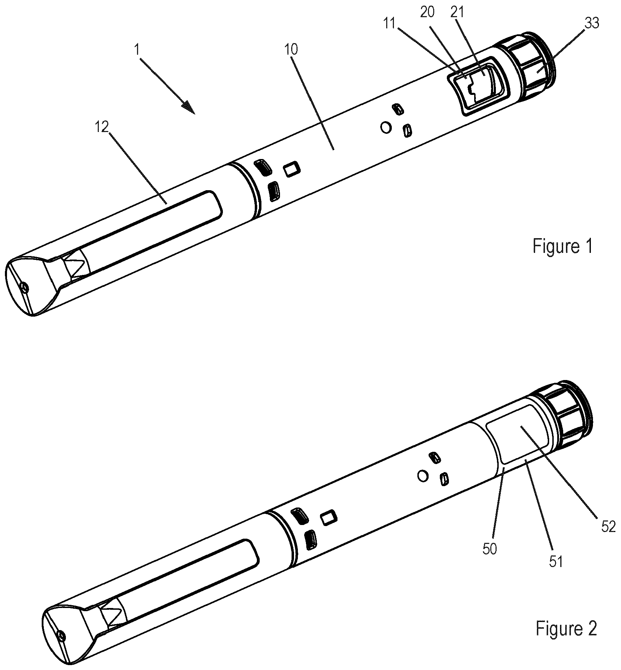

Figure 1 depicts a perspective view of a drug delivery device according to the invention in form of an injection pen 1. In the following description the term distal refers to the side or end of the injection pen where the injection needle is located. This is on the left-hand side in the figures 1 to 3. The term proximal refers to the side or end opposite the needle end. This is on the right-hand side in the figures 1 to 3.

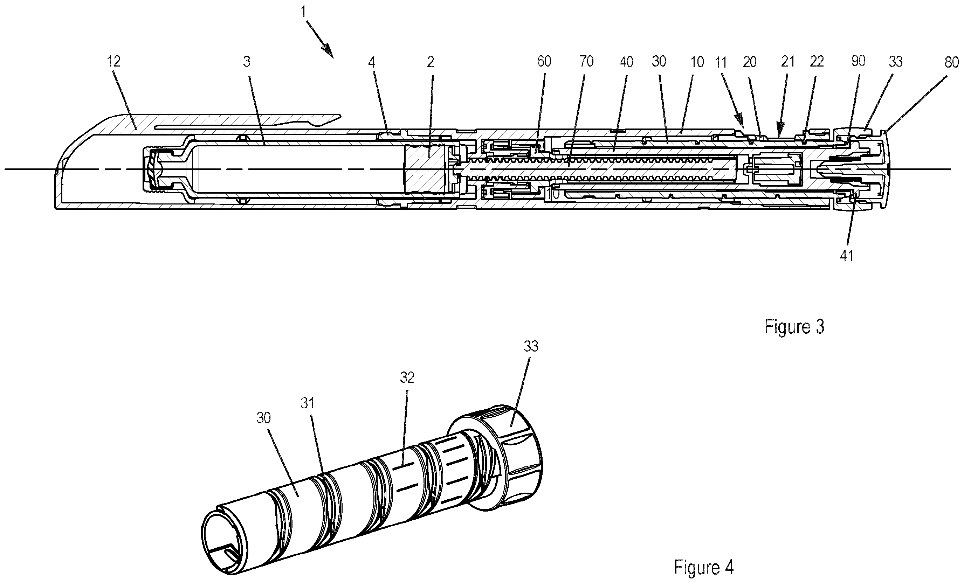

The injection pen 1 comprises an elongated pen housing 10 defining a longitudinal axis. On a distal end a pen cap 12 is located covering an injection needle or a needle mounting portion of the injection pen. The injection pen 1 further includes a cartridge holder 4 holding a cartridge 3 containing the liquid drug, depicted in figure 3.

Figure 3 depicts a sectional view of the injection pen 1. The cut for the sectional view runs along the longitudinal axis of the pen. In the following the most important components of the injection pen and in particular of a dose and dispensing mechanism are described.

The pen housing 10 accommodates the dose and dispensing mechanism. The mechanism includes an indication sleeve 30 (or number sleeve) fixedly connected to a dose knob 33. The indication sleeve 30 together with the dose knob 33 are rotatable relative to the housing 10 to set a dose. The mechanism further includes a sleeve-shaped drive sleeve 40 (or clutch) coaxially arranged inside the indication sleeve 30, a click ring 90 adapted to releasably couple the indication sleeve 30 with the drive sleeve 40, a sleeve-shaped threaded nut 60 coaxially arranged inside the drive sleeve 40 and coupled to the drive sleeve 40 by a spline engagement allowing an axial shifting but preventing a relative rotation between the threaded nut 60 and the drive sleeve 40. Furthermore, the mechanism includes a plunger rod 70 located inside the threaded nut 60 and in threaded connection with the threaded nut 60.

Moreover, the mechanism further comprises a sleeve-shaped threaded insert 20 fixedly connected to the housing 10 such that the insert 20 cannot move relative to the housing 10. In a proximal portion the housing comprises a lateral opening 11 or viewing window in its outer wall (figure 1 and figure 3). The threaded insert 20 includes a second lateral opening 21 matching with the housing opening 11 thereby allowing a user to inspect numbers 32 or markings printed on an outer surface of the indication sleeve 30 from outside through the housing opening 11 and insert opening 21.

In its inside the sleeve-shaped threaded insert 20 includes an internal thread 22 (figure 3) which cooperates with an external thread 31 (see figure 4) of the indication sleeve 30.

To set a dose the user grabs the dose knob 33 and rotates the dose knob 33 relative to the housing 10. This rotates the indication sleeve 30 too and thus screws the indication sleeve 30 and dose knob 33 proximally out of the housing. The user can adjust the dose by inspecting the numbers 32 through the lateral housing opening 11 and insert opening 21. During dose setting proximally oriented saw teeth of the click ring 90 slide over distally oriented saw teeth of the indication sleeve 30 thereby generating a tactile and acoustic feedback signal to the user. During dose setting the drive sleeve 40 is linearly moved in proximal direction as it abuts the indication sleeve 30 via ledge 41 (shown in figure 3) that engages a step inside the indication sleeve 30. As the drive sleeve 40 is not rotationally coupled to the indication sleeve 30 the drive sleeve 40 is axially moved but is not rotated rotate during dose setting and dose correction.

Once the correct dose is set the user presses the button 80 which causes the indication sleeve 30 to move distally in longitudinal direction. This couples the proximal saw teeth of the click ring 90 to the distal saw teeth of the indication sleeve 30 and thus rotationally couples the drive sleeve 40 to the indication sleeve 30. When the user keeps pressing the button 80 the indication sleeve 30 is screwed back into the housing and due to the rotational coupling via click ring 90 the drive sleeve 40 is rotated too.

Due to the spline engagement the threaded nut 60 is rotated together with the drive sleeve 40. As the threaded nut 60 is threadedly connected to the plunger rod 70 and as the plunger rod 70 is splined to the housing 10 the plunger rod 70 is axially shifted in distal direction but is prevented from being rotated relative to the housing 10. A flange at a distal end of the plunger rod 40 moves a piston 2 within the cartridge 3 and thereby dispenses the liquid through the injection needle.

In a first arrangement as shown in figure 2 the injection pen 1 additionally includes a flexible label 50 non-removably bonded or glued on an outer surface of the housing 10 in the area of the opening 11. A magnifying lens in form of a Fresnel lens 52 is integrally formed in the label. The Fresnel lens 52 is formed by a plurality of lens section to provide the Fresnel effect.

Figure 2 shows a perspective view of the injection pen 1 of figure 1 with the flexible label 50 on its outside. As shown in figure 2 the label 50 comprises a magnifying section with the Fresnel lens 52 in a middle portion which is positioned above the housing opening 11 such that the Fresnel lens 52 covers the opening 11. When inspecting the indication sleeve 30 the user sees the numbers 32 of the indication sleeve 30 through the Fresnel lens 52. The label 50 comprises further a bonding section 51 on both sides of the integrally formed lens 52. The bonding section enables to bond or glue the label 50 to the housing surface. The lens 52 and the bonding section 51 are monolithic and thus formed as a one-piece and the label has a constant thickness across its length. The label 50 is made of a flexible plastic material.

The Fresnel lens magnifies the numbers or markings 32 on the indication sleeve 30. In figure 4 the indication sleeve 30 is depicted alone. In this figure the numbers 32 are schematically depicts as markings but usually the indication sleeve comprises a row of circumferentially positioned numbers indicating dose values, for example from 1 to 300 units. In order be able to arrange all numbers on the sleeve they have to be printed in a compressed design in the longitudinal direction. The Fresnel lens 52 has a longer extension along the longitudinal axis than in a direction perpendicular to the longitudinal axis (or in circumferential direction). Therefore, the compressed numbers appear in a normal, non-compressed design when seen through the Fresnel lens 52.

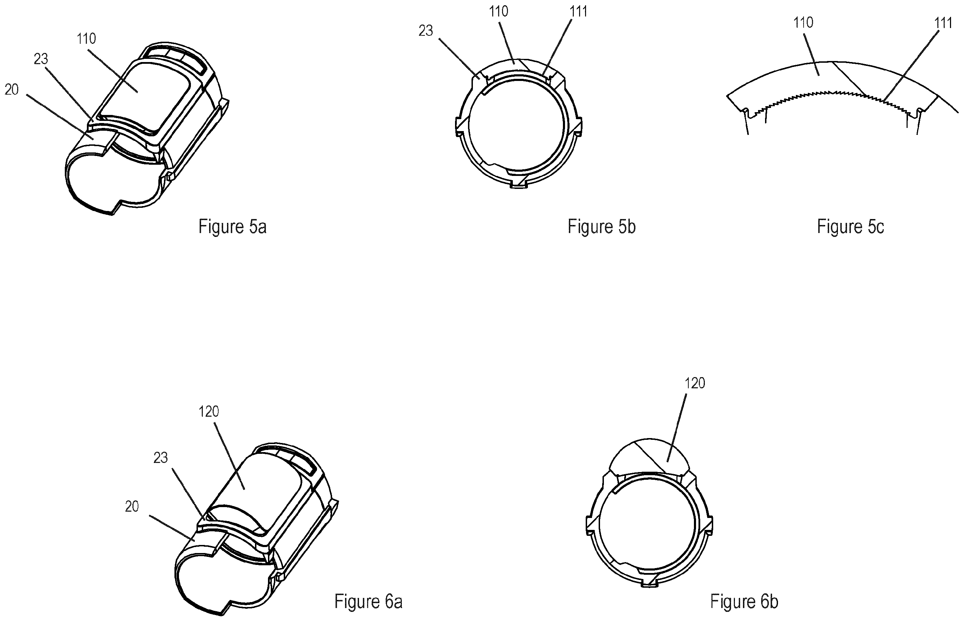

Figure 5a depicts the threaded insert 20 used in a second arrangement. Unlike the first arrangement the injection pen of the second arrangement does not comprise a flexible label with a lens on its outside. Instead, the injection pen includes a Fresnel lens 110 connected to the threaded insert 20 as shown in figures 5a, 5b and 5c.

As best shown in figure 5a a body of the Fresnel lens 110 is held by a holding collar 23 circumferentially arranged around the opening of the threaded insert. The lens 110 is snapped in and held by the collar 23 by form fit as well as by a force fit. Figure 5b depicts a sectional view wherein the cut for the sectional view runs through a plane perpendicular to the longitudinal axis. As it can be seen in figure 5b the Fresnel lens 110 has an arc-shaped geometry in a cross-section. A maximum radial dimension of the lens 110 measured from the central longitudinal axis to an outer surface of the lens is equal to a maximum radial dimension of the collar 23 of the threaded insert. Hence, the maximum radial dimension of the lens 110 from the central axis is equal or less than a minimal radial dimension from the central axis to an inner surface of the hosing. That means an outer surface of the lens 110 does not protrude from the collar 23. This is advantageous as the insert 20 and the housing does not have to be modified for retrofitting the lens. For the assembly, the insert 20 with the Fresnel lens 110 can be inserted into to the housing 10 and connected thereto.

On its outside the Fresnel lens 110 has a smooth surface whereas on its inside the lens comprises a sectional and structured surface 111 comprising saw teeth-shaped lens sections or segments to provide the Fresnel effect. Figure 5c depicts an enlarged view of the Fresnel lens 110 showing the structured surface 111 with the lens sections oriented towards the inside or center.

Similar to the first arrangement the Fresnel lens 110 may have a longer extension along the longitudinal axis than in a direction perpendicular to the longitudinal axis and thus has a stronger magnification in the longitudinal direction than in the radial direction. Therefore, numbers or markings in a compressed design appear in a non-compressed design when seen through the Fresnel lens.

In a further embodiment the insert may be made by two-component injection molding comprising a first material for the body and internal thread of the threaded insert 20 and a second material for the lens 110. In this case the lens is integrally and monolithic formed in the insert 20 as a one-piece.

In an alternative embodiment the Fresnel lens can be integrated directly in the housing 10 and namely in the opening 11 of the housing. In this embodiment the thread for the dose sleeve may be directly on an inside of the housing without a threaded insert.

Figure 6a and 6b depict a further embodiment. In contrast to the embodiment shown in figures 5a, 5b and 5c the lens 120 is not a Fresnel lens but a conventional lens 120 with a bulky body. The lens 120 is held in place by the circumferential collar 23 as described above. As best shown in the cross section in figure 6b the outer radial dimension of the lens 120 clearly exceeds the maximum radius of the threaded insert 20. That means in this embodiment the housing has to be adapted and in particular the opening in the sleeve-shaped housing has to be enlarged such that the insert 20 with the bulky lens 120 can be inserted for assembly.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | |
|---|---|---|---|
| 1 | injection pen | 110 | Fresnel Lens |
| 2 | piston | 111 | Lens sections |
| 3 | cartridge | 210 | conventional lens |
| 4 | cartridge holder | | |
| 10 | housing | | |
| 11 | first lateral opening | | |
| 20 | threaded insert | | |
| 21 | second lateral opening | | |
| 22 | internal thread | | |
| 23 | collar | | |
| 30 | indication sleeve | | |
| 31 | external thread | | |
| 32 | number | | |
| 33 | dose knob | | |
| 40 | drive sleeve | | |
| 50 | flexible label | | |
| 51 | bonding section | | |
| 52 | lens section/lens | | |
| 60 | threaded nut | | |
| 70 | plunger rod | | |
| 80 | button | | |
| 90 | click ring | | |

## Claims

1. A dose setting mechanism for a drug delivery device (1) for dispensing a liquid drug from a reservoir (3) through an injection needle or cannula, wherein the dose setting mechanism is adapted to set a dose to be dispensed from the reservoir (3) and comprising
- a sleeve-shaped housing (10) defining a longitudinal direction and having a central axis and comprising a first lateral opening (11);
- a threaded insert (20) arranged inside the housing (10) and comprising a second lateral opening (21) matching with the first lateral opening (11) and wherein the insert (20) is non-movably connected to the housing (10) and comprises an internal thread (22) on an inner side of the insert;
- an indication member (30) comprising an indication (32) on an outer surface representing a dose value, wherein the indication (32) is visible from outside through the first and second lateral opening (11, 21) and wherein the indication member (30) comprises an outer thread (31) adapted to cooperate with the internal thread (22) of the insert;
- a magnifying lens (52) adapted to provide a magnified view of the indication (32), **characterized in that** the magnifying lens (52) is positioned in the second lateral opening (21) and integrated in the insert (20) or enclosed by the insert (20), wherein a maximum radial dimension measured from the central axis to an outer surface of the magnifying lens (52) is equal or less than a minimum radial dimension from the central axis to an inner surface of the housing (10).

2. Dose setting mechanism according to claim 1, wherein the magnifying lens (52) has an arched-shaped outer contour.

3. Dose setting mechanism according to claim 1 or 2, wherein the threaded insert (20) comprises a holding portion (23) integrally formed in the insert (20) in the area of the second lateral opening (21) and adapted to hold the magnifying lens (52) in place.

4. Dose setting mechanism according to claim 3, wherein the holding portion (23) is a collar at least partially encompassing the magnifying lens (52) such that the magnifying lens is axially and radially held in place by a form fit.

5. Dose setting mechanism according to claim 1 or 2, wherein the threaded insert (20) is made by multi-material injection moulding wherein a first component is used for the internal thread (22) and the body of the insert and a second component is used for the magnifying lens (52).

6. Dose setting mechanism according to any of claims 1 to 5, wherein the magnifying lens (52) is a Fresnel lens comprising a plurality of lens sections.

7. Dose setting mechanism according to any of claim 1 to 6, wherein the magnifying lens (52) comprises a planar surface and an uneven surface, wherein the uneven surface is directed towards the indication member (30).

8. Dose setting mechanism according to any of claims 1 to 7, wherein a dimension of the magnifying lens (52) along the longitudinal axis is larger than a dimension of the lens (52) in a direction perpendicular to the longitudinal axis.

9. Dose setting mechanism according to any of claims 1 to 8, wherein the indication member (30) is a sleeve rotatable relative to the housing (10) for dose setting.

10. Dose setting mechanism according to any of claims 1 to 9, wherein the housing (10) is the outermost part such that the housing envelops the indication member (30) and wherein the housing (10) is further adapted to envelop a dispensing mechanism of the delivery device.

11. A delivery device comprising the dose setting mechanism according to any of claims 1 to 10 and further comprising a reservoir holder (4) attachable to the housing (10) and adapted to hold the reservoir (3) relative to the housing.

12. Delivery device according to claim 11 comprising a dispensing mechanism for dispensing the set dose, wherein the dispensing mechanism comprises a dose set member (33) rotatable relative to the housing (10) to set a dose, a drive member (40) that can be coupled or decoupled to the dose set member (33) and a plunger rod (70) operatively coupled to the drive member (40), wherein during dose setting the drive member (40) is not coupled to the dose set member (33) and during dispensing the dose set member (33) is coupled to the driver member (40) to transmit a rotation of the dose set member (33) to the driver member to move the plunger rod (70) in dispensing direction.

13. Delivery device according to claim 11 or 12, wherein the device (1) is a pen-shaped injection device and wherein the reservoir holder (4) includes a connecting portion adapted to releasably attach an injection needle and wherein the reservoir (3) is a cartridge.
